(19) Europäisches Patentamt
European Patent Office
Office européen des brevets

(11) Veröffentlichungsnummer: **0 307 762**
**A1**

(12) **EUROPÄISCHE PATENTANMELDUNG**

(21) Anmeldenummer: **88114520.5**

(22) Anmeldetag: **06.09.88**

(51) Int. Cl.⁴: **C07D 295/18 , C07C 103/58 , A01N 43/84 , A01N 43/40 , A01N 37/18**

Patentansprüche für folgenden Vertragsstaat: ES.

(30) Priorität: **16.09.87 CH 3570/87**
**13.07.88 CH 2671/88**

(43) Veröffentlichungstag der Anmeldung:
**22.03.89 Patentblatt 89/12**

(84) Benannte Vertragsstaaten:
**AT BE CH DE ES FR GB GR IT LI NL**

(71) Anmelder: **F. HOFFMANN-LA ROCHE & CO. Aktiengesellschaft**

**CH-4002 Basel(CH)**

(72) Erfinder: **Ziegler, Hugo, Dr.**
**Oberwilerstrasse 39**
**CH-4123 Allschwil(CH)**
Erfinder: **Zurflüh, René, Dr.**
**Dachslenbergstrasse 54**
**CH-8180 Bülach(CH)**

(74) Vertreter: **Urech, Peter, Dr. et al**
**Grenzacherstrasse 124 Postfach 3255**
**CH-4002 Basel(CH)**

(54) **Acrylsäureamide und ihre Verwendung als Fungizide.**

(57) Die Erfindung betrifft Verbindungen der Formel

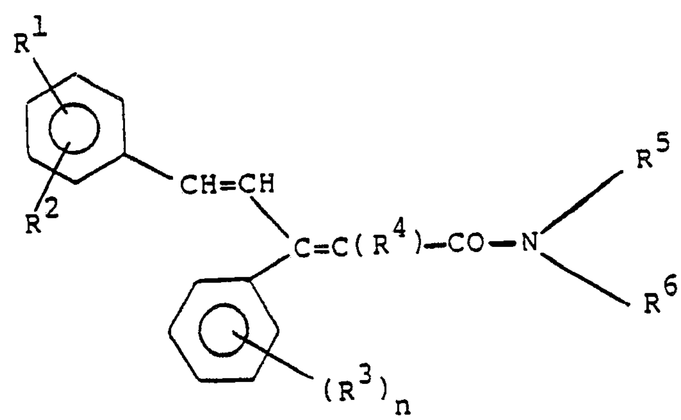

I

worin $R^1$, $R^2$, $R^3$, $R^4$, $R^5$, $R^6$ und n die in der Beschreibung angegebenen Bedeutungen besitzen, Verfahren zu deren Herstellung, fungizide Mittel, die diese Verbindungen als Wirkstoffe enthalten, sowie die Verwendung solcher Verbindungen bzw. Mittel zur Bekämpfung von Fungi in der Landwirtschaft und im Gartenbau.

EP 0 307 762 A1

Xerox Copy Centre

## Acrylsäureamide und ihre Verwendung als Fungizide

Die vorliegende Erfindung betrifft neue Verbindungen, und zwar Acrylsäureamide der allgemeinen Formel

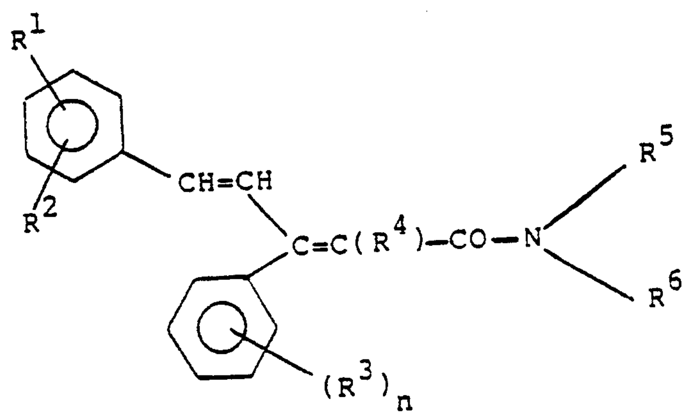

I

worin
$R^1$ $C_{1-6}$-Halogenalkyl, $C_{2-6}$-Halogenalkenyl, $C_{1-6}$-Halogenalkoxy, $C_{2-6}$-Halogenalkenyloxy, $C_{1-6}$-Alkylthio, $C_{1-6}$-Halogenalkylthio, Cyano, gegebenenfalls substituiertes Phenyl oder gegebenenfalls substituiertes Phenoxy,
$R^2$ Wasserstoff oder Halogen,
$R^3$ Halogen, $C_{1-6}$-Alkyl oder $C_{1-6}$-Alkoxy,
$R^4$ Wasserstoff oder $C_{1-6}$-Alkyl,
$R^5$ und $R^6$ unabhängig voneinander $C_{1-4}$-Alkyl oder zusammen mit dem Stickstoffatom, an das sie geknüpft sind, eine Morpholino- oder Thiomorpholinogruppe und
n 1, 2 oder 3 bedeuten.

Die Verbindungen der Formel I besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der Verbindungen der Formel I, fungizide Mittel, die solche Verbindungen als Wirkstoffe enthalten, sowie die Verwendung solcher Verbindungen und Mittel zur Bekämpfung von Fungi in der Landwirtschaft und im Gartenbau.

In der obigen Formel I umfasst der Ausdruck "Halogen" Fluor, Chlor, Brom oder Jod, wobei Fluor, Chlor und Brom bevorzugt sind. Die Gruppen Halogenalkyl, Halogenalkenyl, Halogenalkoxy, Halogenalkenyloxy und Halogenalkylthio können jeweils einen oder mehrere (gleiche oder verschiedene) Halogensubstituenten aufweisen.

Die gegebenenfalls mit Halogen substituierten Alkyl-, Alkenyl-, Alkoxy-, Alkenyloxy und Alkylthiogruppen können geradkettig oder verzweigt sein. Die substituierten Phenyl- und Phenoxygruppen weisen insbesondere bis 2 (gleiche oder verschiedene) Substituenten auf, die vorzugsweise aus Halogen, $C_{1-4}$-Alkyl und $C_{1-4}$-Halogenalkyl ausgewählt sind. Falls n 2 oder 3 bedeutet, ist das eine Symbol $R^3$ unabhängig vom anderen Symbol $R^3$.

Das Vorhandensein mindestens eines asymmetrischen Kohlenstoffatoms in den erfindungsgemässen Verbindungen hat zur Folge, dass die Verbindungen als optische Antipoden auftreten können. Ferner können die Verbindungen als E- und Z-Isomere und/oder als Atropisomere vorliegen. Die Formel I soll all diese möglichen isomeren Formen und deren Gemische umfassen.

Unabhängig voneinander bedeuten $R^1$ vorzugsweise $C_{1-6}$-Halogenalkyl, insbesondere Trifluormethyl, oder $C_{1-6}$-Halogenalkoxy, insbesondere Trifluormethoxy; $R^3$ vorzugsweise $C_{1-4}$-Alkoxy, insbesondere Methoxy; und $NR^5R^6$ vorzugsweise Aethylmethylamino, Morpholino oder Thiomorpholino. Der bzw. jeder Substituent $R^3$ befindet sich vorzugsweise in der m- oder p-Stellung.

Besonders bevorzugte einzelne Verbindungen der Formel I sind:
4-[3-(3,4-Dimethoxyphenyl)-5-(α,α,α-trifluor-p-tolyl)-2,4-pentadienoyl]-morpholin,
4-[3-(3,4-Dimethoxyphenyl)-5-(α,α,α-trifluor-p-tolyl)-2,4-pentadienoyl]-thiomorpholin und

N-Aethyl-N-methyl-3-(3,4-dimethoxyphenyl)-5-(α,α,α-trifluor-p-tolyl)-2,4-pentadiensäureamid.

Weitere Vertreter von Verbindungen der Formel I sind:

4-[5-(3-Cyanophenyl)-3-(3,4-dimethoxyphenyl)-2,4-pentadienoyl]-morpholin,
4-[3-(3,4-Dimethoxyphenyl)-5-(α,α,α-trifluor-m-tolyl)-2,4-pentadienoyl]-morpholin,
4-[5-(4-Biphenylyl)-3-(3,4-dimethoxyphenyl)-2,4-pentadienoyl]-morpholin,
4-{3-(3,4-Dimethoxyphenyl)-5-[4-(2,2,2-trifluoräthoxy)-phenyl)-2,4-pentadienoyl}-morpholin,
4-{5-[4-(n-Butylthio)-phenyl]-3-(3,4-dimethoxyphenyl)-2,4-pentadienoyl}-morpholin,
4-[3-(3,4-Dimethoxyphenyl)-5-(4-fluor-α,α,α-trifluor-m-tolyl)-2,4-pentadienoyl]-morpholin,
4-[3-(3,4-Dimethoxyphenyl)-5-(3-phenoxyphenyl)-2,4-pentadienoyl]-morpholin,
4-{5-[4-(4-Chlorphenoxy)-phenyl]-3-(3,4-dimethoxyphenyl)-2,4-pentadienoyl}-morpholin,
4-{3-(3,4-Dimethoxyphenyl)-5-[4-(α,α,α-trifluor-p-tolyloxy)-phenyl]-2,4-pentadienoyl}-morpholin,
4-[3-(3-Aethyl-4-methoxyphenyl)-5-(α,α,α-trifluor-p-tolyl)-2,4-pentadienoyl]-morpholin,
4-[3-(4-Aethoxyphenyl)-5-(α,α,α-trifluor-p-tolyl)-2,4 pentadienoyl]-morpholin,
4-[3-(3,4-Diäthoxyphenyl)-5-(α,α,α-trifluor-p-tolyl)-2,4-pentadienoyl]-morpholin,
4-[3-(4-Methoxy-m-tolyl)-5-(α,α,α-trifluor-p-tolyl)-2,4-pentadienoyl]-morpholin,
4-[3-(3,4-Dimethylphenyl)-5-(α,α,α-trifluor-p-tolyl)-2,4-pentadienoyl]-morpholin,
4-[3-(3-Aethoxy-4-methoxyphenyl)-5-(α,α,α-trifluor-p-tolyl)-2,4-pentadienoyl]-morpholin,
4-[3-(4-Aethoxy-3-methoxyphenyl)-5-(α,α,α-trifluor-p-tolyl)-2,4-pentadienoyl]-morpholin,
4-[3-(4-Methoxy-3-propoxyphenyl)-5-(α,α,α-trifluor-p-tolyl)-2,4-pentadienoyl]-morpholin,
4-[3-(3-Chlor-4-methoxyphenyl)-5-(α,α,α-trifluor-p-tolyl)-2,4-pentadienoyl]-morpholin,
4-{5-[m-(1,2-Dichlorvinyloxy)-phenyl]-3-(3,4-dimethoxyphenyl)-2,4-pentadienoyl]-morpholin,
4-[3-(4-Methoxy-m-tolyl)-5-(α,α,α-trifluor-m-tolyl)-2,4-pentadienoyl]-morpholin,
4-[3-(3-Aethoxy-4-methoxyphenyl)-5-(α,α,α-trifluor-m-tolyl)-2,4-pentadienoyl]-morpholin,
N-Aethyl-N-methyl-3-(4-methoxy-m-tolyl)-5-(α,α,α-trifluor-p-tolyl)-2,4-pentadiensäureamid,
N-Aethyl-N-methyl-3-(3,4-dimethylphenyl)-5-(α,α,α-trifluor-p-tolyl)-2,4-pentadiensäureamid,
N-Aethyl-N-methyl-3-(3-äthoxy-4-methoxyphenyl)-5-(α,α,α-trifluor-p-tolyl)-2,4-pentadiensäureamid,
N-Aethyl-N-methyl-3-(4-äthoxy-3-methoxyphenyl)-5- (α,α,α-trifluor-p-tolyl)-2,4-pentadiensäureamid,
N-Aethyl-N-methyl-3-(4-methoxy-3-propoxyphenyl)-5-(α,α,α-trifluor-p-tolyl)-2,4-pentadiensäureamid,
N-Aethyl-N-methyl-3-(3-chlor-4-methoxyphenyl)-5-(α,α,α-trifluor-p-tolyl)-2,4-pentadiensäureamid,
N-Aethyl-N-methyl-3-(3-äthoxy-4-methoxyphenyl)-5-(α,α,α-trifluor-m-tolyl)-2,4-pentadiensäureamid,.
N-Aethyl-N-methyl-3-(4-methoxy-3-propoxyphenyl)-5-(α,α,α-trifluor-m-tolyl)-2,4-pentadiensäureamid,
N-Aethyl-N-methyl-3-(3-chlor-4-methoxyphenyl)-5-(α,α,α-trifluor-m-tolyl)-2,4-pentadiensäureamid,
N-Aethyl-N-methyl-2-methyl-3-(4-methoxy-m-tolyl)-5-(α,α,α-trifluor-p-tolyl)-2,4-pentadiensäureamid,
N-Aethyl-N-methyl-2-methyl-3-(3,4-dimethylphenyl)-5-(α,α,α-trifluor-p-tolyl)-2,4-pentadiensäureamid,
N-Aethyl-N-methyl-2-methyl-3-(3-äthoxy-4-methoxyphenyl)-5-(α,α,α--trifluor-p-tolyl)-2,4-pentadiensäureamid,
N-Aethyl-N-methyl-2-methyl-3-(4-äthoxy-3-methoxyphenyl)-5-(α,α,α-trifluor-p-tolyl)-2,4-pentadiensäureamid,
N-Aethyl-N-methyl-2-methyl-3-(4-methoxy-3-propoxyphenyl)-5-(α,α,α-trifluor-p-tolyl)-2,4-pentadiensäureamid,
N-Aethyl-N-methyl-2-methyl-3-(3-chlor-4-methoxyphenyl)-5-(α,α,α-trifluor-p-tolyl)-2,4-pentadiensäureamid,
4-[3-(3,4-Dimethoxyphenyl)-2-methyl-5-(α,α,α-trifluor-m-tolyl)-2,4-pentadienoyl]-morpholin,
4-[3-(3,4-Dimethoxyphenyl)-2-methyl-5-(α,α,α-trifluor-p-tolyl)-2,4-pentadienoyl]-thiomorpholin,
4-[3-(3,4-Dimethoxyphenyl)-2-methyl-5-(α,α,α-trifluor-m-tolyl)-2,4-pentadienoyl]-thiomorpholin,
4-[3-(4-Methoxy-m-tolyl)-5-(α,α,α-trifluor-p-tolyl)-2,4-pentadienoyl]-thiomorpholin,
4-[3-(3,4-Dimethylphenyl)-5-(α,α,α-trifluor-p-tolyl)-2,4-pentadienoyl]-thiomorpholin,
4-[3-(3-Aethoxy-4-methoxyphenyl)-5-(α,α,α-trifluorp-tolyl)-2,4-pentadienoyl]-thiomorpholin,
4-[3-(4-Aethoxy-3-methoxyphenyl)-5-(α,α,α-trifluor-p-tolyl)-2,4-pentadienoyl]-thiomorpholin,
4-[3-(4-Methoxy-3-propoxyphenyl)-5-(α,α,α-trifluor-p-tolyl)-2,4-pentadienoyl]-thiomorpholin,
4-[3-(3-Chlor-4-methoxyphenyl)-5-(α,α,α-trifluor-p-tolyl)-2,4-pentadienoyl]-thiomorpholin,
4-[3-(3,4-Dimethoxyphenyl)-5-(α,α,α-trifluor-m-tolyl)-2,4-pentadienoyl]-thiomorpholin und
4-[3-(3-Aethoxy-4-methoxyphenyl)-5-(α,α,α-trifluor-m-tolyl)-2,4-pentadienoyl]-thiomorpholin.

Das erfindungsgemässe Verfahren zur Herstellung der Verbindungen der Formel I ist dadurch gekennzeichnet, dass man

a) ein Keton der allgemeinen Formel

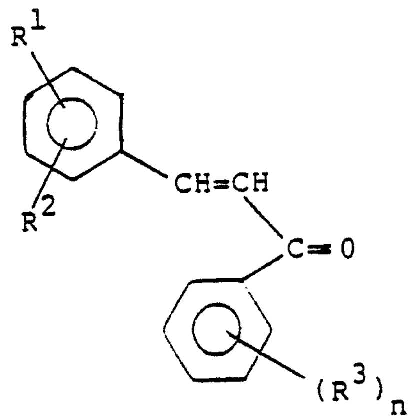

II

worin $R^1$, $R^2$ $R^3$ und n die oben angegebenen Bedeutungen besitzen, mit einem Phosphonoalkansäureamaid der allgemeinen Formel

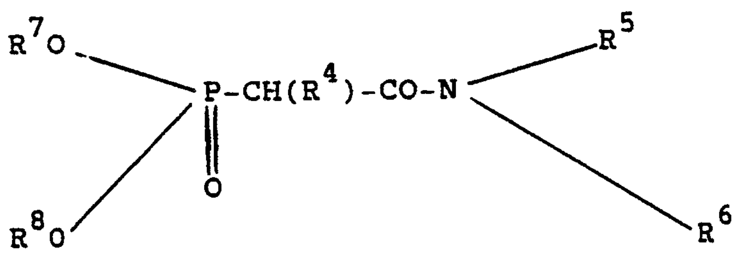

III

worin $R^4$, $R^5$ und $R^6$ die oben angegebenen Bedeutungen besitzen und $R^7$ und $R^8$ unabhängig voneinander eine Niederalkylgruppe oder Arylgruppe bedeuten, umsetzt, oder

b) eine Acrylsäure der allgemeinen Formel

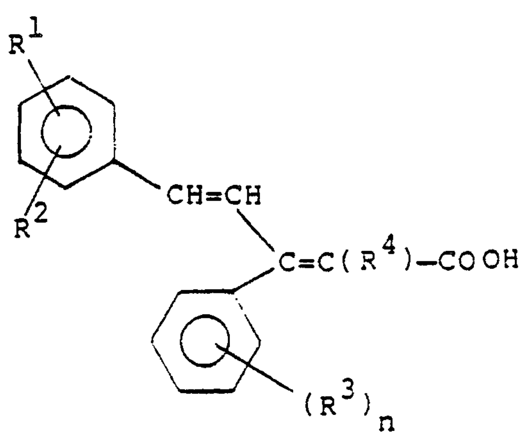

IV

worin $R^1$, $R^2$, $R^3$, $R^4$ und n die oben angegebenen Bedeutungen besitzen, oder ein reaktionsfähiges Derivat davon mit einem Amin der allgemeinen Formel

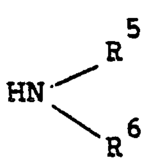

V

worin $R^5$ und $R^6$ die oben angegebenen Bedeutungen besitzen, oder mit einem reaktionsfähigen Derivat davon umsetzt.

Bei der Verfahrensvariante a) handelt es sich um eine Horner-Wittig-Reaktion, die unter den diesbezüglich üblichen Reaktionsbedingungen durchgeführt werden kann. Unter die im Phosphonoalkansäureamid der Formel III vorhandenen Niederalkyl- bzw. Arylgruppe $R^7$ oder $R^8$ ist vorzugsweise eine $C_{1-4}$-Alkylgruppe, insbesondere eine Methyl- oder Aethylgruppe, bzw. eine gegebenenfalls mit Chlor und/oder Methyl substituierte Phenylgruppe zu verstehen. Die Umsetzung erfolgt zweckmässigerweise in einem inerten organischen Verdünnungsmittel, wie einem aromatischen Kohlenwasserstoff, z.B. Benzol oder Toluol, einem aliphatischen oder cyclischen Aether, z.B. 1,2-Dimethoxyäthan, Tetrahydrofuran oder Dioxan, oder einem Dialkylamid, z.B. Dimethylformamid, und in Gegenwart einer Base, wie eines Alkalimetallhydrids, z.B. Natriumhydrid, eines Alkalimetallalkoholats, z.B. Natriummethylat oder Kalium-tert.butylat, oder eines Alkalimetallamids, z.B. Natriumamid, bei Temperaturen zwischen -20°C und der Rückflusstemperatur des Reaktionsgemisches, vorzugsweise imTemperaturbereich von -0°C bis 80°C. Falls ein Alkalimetallalkoholat als Base verwendet wird, erfolgt die Umsetzung zweckmässigerweise in einem dem jeweiligen Alkalimetallalkoholat entsprechenden Alkohol als Verdünnungsmittel. Weitere Hinweise auf geeignete Reaktionsbedingungen für die Horner-Wittig Reaktion finden sich beispielsweise in Houben-Weyl, Methoden der Organischen Chemie, Band V/Ib, Seiten 395-400 und 895-899 und in Synthesis 1974, Seiten 122-124.

Die Umsetzung nach Verfahrensvariante b) stellt eine Acylierung dar und kann unter den üblichen Reaktionsbedingungen durchgeführt werden. Als reaktionsfähige Derivate der Acrylsäuren der Formel IV kommen insbesondere ihre Säurehalogenide, insbesondere die Säurechloride; Säureanhydride; gemischte Säureanhydride, insbesondere diejenigen mit aliphatischen oder aromatischen Carbon- oder Sulfonsäuren; Niederalkyl-, Aryl- oder Benzylester; sowie Imidazolide in Frage. Solche Derivate können auch in situ erzeugt werden. Als Alternative zur Verwendung des reaktionsfähigen Derivats kann die Acylierung in Gegenwart eines säureaktivierenden Mittels oder eines wasserentziehenden Mittels durchgeführt werden, wie beispielsweise, N,N-Carbonyldiimidazol, N,N-Dicyclohexylcarbodiimid oder eines Chlorameisensäureesters. Diese genannten Mittel erfüllen beide Rollen. Zu den reaktionsfähigen Derivaten der Amine der Formel V gehören insbesondere ihre Phosphazo-Derivate. Man setzt in der Regel eine Acrylsäure IV mit einem Amin V in Gegenwart eines säureaktivierenden oder wasserentziehenden Mittels, oder ein reaktionsfähiges Derivat der Acrylsäure mit dem Amin, oder die Acrylsäure mit einem reaktionsfähigen Derivat des Amins um.

Zudem erfolgt die Umsetzung zweckmässigerweise in Gegenwart eines inerten organischen Verdünnungsmittels, wie eines aromatischen Kohlenwasserstoffes, z.B. Benzol oder Toluol; eines aliphatischen oder cyclischen Aethers, z.B. Diäthyläther, tert.Butylmethyläther, Tetrahydrofuran oder Dioxan; eines halogenierten aliphatischen Kohlenwasserstoffes, z.B. Methylenchlorid oder 1,2-Dichloräthan; Acetonitril; oder eines Dialkylamids, z.B. Dimethylformamid, bei Temperaturen zwischen -20°C und 100°C, vorzugsweise zwischen -10°C und 50°C. Gegebenenfalls wird in Gegenwart eines Säurebindemittels, wie einer anorganischen Base, z.B. Kaliumcarbonat, oder einer organischen Base, z.B. Triäthylamin, Pyridin oder Chinolin, gearbeitet, wobei jede der genannten organischen Basen gleichzeitig als Lösungsmittel dienen kann.

Weitere Hinweise auf geeignete Reaktionsbedingungen für die Acylierung finden sich beispielsweise in J. Zabicky, The Chemistry of Amides, Seite 73 ff. (Interscience 1970).

Die Isolierung und Reinigung der so hergestellten Verbindungen der Formel I können nach an sich bekannten Methoden erfolgen. Ebenfalls nach an sich bekannten Methoden können allfällig erhaltene Isomerengemische, z.B. E/Z-Isomerengemische, in die reinen Isomeren aufgetrennt werden, beispielsweise durch Chromatographie oder fraktionierte Kristallisation.

Die als Ausgangsmaterialien der Verfahrensvariante a) verwendeten Ketone der Formel II sind entweder bekannt oder können nach an sich bekannten Methoden hergestellt werden, beispielsweise durch Kondensation eines substituierten Acetophenons der allgemeinen Formel

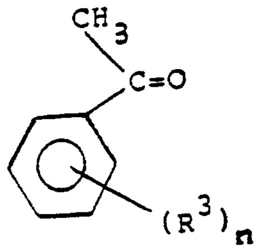

VI

worin $R^3$ und n die oben angegebenen Bedeutungen besitzen,
mit einem substituierten Benzaldehyd der allgemeinen Formel

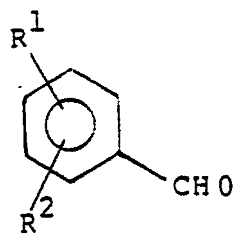

VII

worin $R^1$ und $R^2$ die oben angegebenen Bedeutungen besitzen,
zweckmässigerweise unter Verwendung eines basischen Kondensationsmittels, wie eines Alkali- oder Erdalkalimetallhydroxids, z.B. Natrium-, Kalium-, Calcium- oder Bariumhydroxid, eines $C_{1-4}$-Alkanolats, z.B. Natriummethanolat, Natriumäthanolat, Kalium-tert.butanolat oder Aluminiumtert.butanolat, Natriumacetat, Pyrrolidin oder Piperidin, oder unter Verwendung eines sauren Katalysators, wie einer Mineralsäure, z.B. Salzsäure, oder Phosphoroxychlorid oder Zinkchlorid. Die Verwendung eines Verdünnungsmittels ist nicht zwingend, jedoch bevorzugt. Als Verdünnungsmittel eignen sich insbesondere solche inerte Verdünnungsmittel wie niedere Alkohole, z.B. Methanol, Aethanol und tert.Butanol. Die Kondensation erfolgt in der Regel bei Temperaturen zwischen 0°C und der Rückflusstemperatur des Reaktionsgemisches, vorzugsweise bei Raumtemperatur.

Unter den neuen Ketonen der Formel II stellt das 1-(3,4-Dimethoxyphenyl)-3-(α,α,α-trifluor-p-tolyl) -2-propen-1-on ein wichtiges Ausgangsmaterial dar.

Auch die als Ausgangsmaterialien der Verfahrensvariante a) verwendeten Phosphonoalkansäureamide der Formel III sind entweder bekannt oder nach an sich bekannten Methoden herstellbar. Beispielsweise kann man ein Amin der oben angegebenen Formel V mit einem α-Chlor- oder α-Bromalkansäurechlorid der allgemeinen Formel

$Hal-CH(R^4)-COCl$ VIII

worin $R^4$ die oben angegebene Bedeutung besitzt und Hal Chlor oder Brom bedeutet,
umsetzen, und das daraus resultierende α-Halogenalkansäureamid der allgemeinen Formel

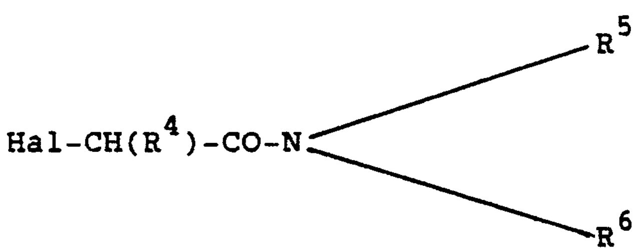

IX

worin $R^4$, $R^5$, $R^6$ und Hal die oben angegebenen Bedeutungen besitzen,
mit einem Phosphit der allgemeinen Formel

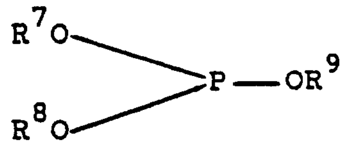

X

worin $R^7$ und $R^8$ die oben angegebenen Bedeutungen besitzen und $R^9$ ebenfalls eine Niederalkyl- oder Arylgruppe bedeutet,
umsetzt. Die Umsetzung der Verbindungen V und VIII miteinander erfolgt zweckmässigerweise in Gegenwart eines inerten organischen Verdünnungsmittels, wie eines aliphatischen oder cyclischen Aethers, z.B. Diäthyläther, Tetrahydrofuran oder Dioxan, oder eines chlorierten aliphatischen Kohlenwasserstoffes, z.B. Methylenchlorid, Aethylenchlorid oder Chloroform, bei Temperaturen zwischen -20° C und 50° C. Gegebenenfalls wird in Gegenwart eines Säurebindemittels, wie einer anorganischen oder organischen Base, gearbeitet, wobei die Verbindung V selber als organische Base dienen kann.

Anschliessend setzt man das α-Halogenalkansäureamid IX mit dem Phosphit X um, und zwar zweckmässigerweise in Gegenwart eines geringen Ueberschusses des Phosphits, welches als Verdünnungsmittel dient. Gegebenenfalls kann ein hochsiedendes inertes Lösungsmittel, z.B. Xylol, verwendet werden. Die Umsetzung erfolgt geeigneterweise in einem Temperaturbereich von 100° C, vorzugsweise bei Temperaturen zwischen 130° C und 160° C.

Die als Ausgangsmaterialien der Verfahrensvariante b) dienenden Acrylsäuren der Formel IV, die ebenfalls entweder bekannt oder in an sich bekannter Weise herstellbar sind, könne beispielsweise dadurch hergestellt werden, dass man ein Keton der oben angegebenen Formel II mit einem Phosphonoalkansäureester der allgemeinen Formel

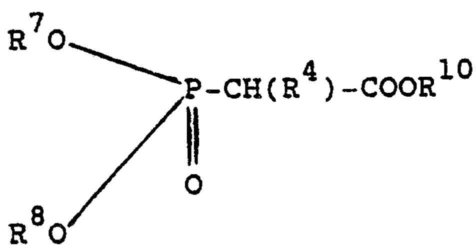

XI

worin $R^4$, $R^7$ und $R^8$ die oben angegebenen Bedeutungen besitzen und $R^{10}$ $C_{1-4}$-Alkyl bedeutet,
umsetzt, und zwar unter den im Zusammenhang mit der Verfahrensvariante a) oben angegebenen Reaktionsbedingungen, worauf der so erhaltene $C_{1-4}$-Alkylester auf konventionelle Weise, beispielsweise unter Verwendung von Natrium- oder Kaliumhydroxid in wässrigem Methanol, zur entsprechenden Säure der Formel IV hydrolysiert wird.

Als weitere Methode zur Herstellung der Acrylsäuren der Formel IV dient die Kondensation eines Acrylsäureesters der allgemeinen Formel

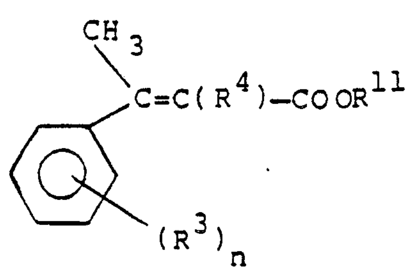

XII

worin $R^3$, $R^4$ und n die oben angegebenen Bedeutungen besitzen
und $R^{11}$ $C_{1-4}$-Alkyl bedeutet,
mit einem substituierten Benzaldehyd der oben angegebenen Formel VII. Diese Kondensation erfolgt zweckmässigerweise unter Verwendung eines basischen Kondensationsmittels, wie eines $C_{1-4}$-Alkanolats, z.B. Kalium-tert. butanolat, das auch noch mit dem bei der Kondensation freiwerdenden Wasser zur

Hydrolyse der Esterfunktion dient und deshalb zumindest in äquimolarer Menge eingesetzt wird. Zudem wird zweckmässigerweise in einem inerten Verdünnungsmittel, wie Dimethylformamid, im Temperaturbereich von 0° C bis 50° C, insbesondere bei Raumtemperatur, gearbeitet.

Ausgehend von den Acrylsäuren IV können deren reaktions- fähige Derivate nach an sich bekannten Methoden hergestellt werden.

Unter den neuen Acrylsäuren der Formel IV bzw. reaktionsfähigen Derivaten davon stellen die 3-(3,4-Dimethoxyphenyl)-5-(α,α,α-trifluor -p-tolyl)-2,4-pentadiensäure und ihre reaktionsfähigen Derivate, z.B. ihre $C_{1-4}$-Alkylester und Alkalimetall-, Erdalkalimetall- und gegebenenfalls substituierten Ammoniumsalze, wichtige Ausgangsmaterialien dar.

Die zur Herstellung der Ausgangsmaterialien der Formeln II, III und IV dienenden Ausgangsmaterialien der Formeln VI-VIII und X-XII sowie die Ausgangsmaterialien der Formel V und deren reaktionsfähige Derivate sind entweder bekannt oder können nach an sich bekannten Methoden hergestellt werden.

Die erfindungsgemässen Verbindungen, d.h. die Verbindungen der Formel I, besitzen fungizide Wirkung und können dementsprechend zur Bekämpfung von Schadpilzen in der Landwirtschaft und im Gartenbau Verwendung finden. Sie können sowohl gegen parasitäre Pilze an Pflanzenteilen, z.B. Blättern, Stengeln, Wurzeln, Zwiebeln, Knollen, Früchten und Blüten, und an Saatgut als auch gegen im Erdboden auftretende phytopathogene Pilze eingesetzt werden. Die erfindungsgemässen Verbindungen eignen sich besonders zur Bekämpfung von Pilzen der zur Klasse der Oomycetes gehörenden Gattungen Plasmopara, Peronospora, Phytophthora, Pseudoperonospora, Bremia und Pythium. Sie sind ferner wirksam unter anderem bei der Bekämpfung von Pilzen der Klassen Deuteromycetes, Ascomycetes und Basidiomycetes, wie beispielsweise Botrytis cinerea, Erysiphe cichoracearum, Erysiphe graminis, Uncinula necator, Podosphaera leucotricha, Venturia inaequalis, Cercosphora archidicola, Cercospora beticola, Helminthosporium gramineum, Cercosporella sp., Septoria sp. und Pellicularia sp.

Die erfindungsgemässen Verbindungen zeichnen sich durch lokale und teilweise auch systemische Wirkung sowie durch eine hohe Pflanzenverträglichkeit aus. Sie können sowohl vorbeugend als auch kurativ angewendet werden.

Unter Gewächshausbedingungen wirken die erfindungsgemässen Verbindungen bereits bei Konzentrationen von 1 mg bis 500 mg Wirkstoff pro Liter Spritzbrühe. Im Freiland werden vorteilhaft Konzentrationen von 50 g bis 1500 g Wirkstoff der Formel I pro Hektar und Behandlung zur Anwendung gebracht. Zur Bekämpfung von samen- oder bodenbürtigen Pilzen im Beizverfahren werden mit Vorteil 0,05 g bis 1,5 g Wirkstoff der Formel I pro kg Samen verwendet.

Die erfindungsgemässen Verbindungen können zu verschiedenartigen Mitteln, z.B. Lösungen, Suspensionen, Emulsionen, emulgierbaren Konzentraten und pulverförmigen Präparaten, formuliert werden. Die erfindungsgemässen fungiziden Mittel sind dadurch gekennzeichnet, dass sie eine wirksame Menge mindestens einer Verbindung der allgemeinen Formel I, wie oben definiert, sowie Formulierungshilfsstoffe enthalten. Die Mittel enthalten zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne Tensidwirkung); und Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30° C und Siedepunkte von mindestens 50° C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäurester und Butylhydroxytoluol; UV-Absorber, z.B. substituierte Benzophenone, Diphenylacrylnitrilsäureester und Zimtsäureester; und Deaktivatoren, z.B. Salze der Aethylendiamintetraessigsäure und Polyglykole.

Die erfindungsgemässen fungiziden Mittel können neben den Wirkstoffen der Formel I auch andere Wirkstoffe enthal ten, z.B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen 0,0001 und 95 Gewichtsprozent an erfindungsgemässer Verbindung bzw. erfindungsgemässen Verbindung als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich des obigen Konzentrationsintervalls. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich des obigen Konzentrationsintervalls. Emulgierbare Konzentrate enthalten im allgemeinen 5 bis 95 Gewichtsprozent, vorzugsweise 25 bis 75 Gewichtsprozent, der Verbindung bzw. Verbindungen der Formel I. Als Anwendungsformen kommen u.a. gebrauchsfertige Lösungen, Emulsionen und Suspensionen, die sich beispielsweise als Spritzbrühen eignen, in Frage. In solchen Spritzbrühen können z.B. Konzentrationen zwischen 0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man mindestens eine Verbindung der allgemeinen Formel I mit Formulierungshilfsstoffen vermischt.

Die Herstellung der Mittel kann in bekannter Weise durchgeführt werden, z.B. durch Vermischen der Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs- bzw. Dispersionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemässe Verbindung in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung.

I. Herstellung der Wirkstoffe der Formel I:

## Beispiel 1

Unter Rühren und Feuchtigkeitsausschluss wird eine Lösung von 4,0 g Diäthylphosphonoessigsäuremorpholid in 50 ml absolutem 1,2-Dimethoxyäthan portionenweise mit 1,7 g Kalium-tert.butylat versetzt. Man erwärmt das Reaktionsgemisch 2 Stunden bei 50 °C und gibt dann 3,4 g (E)-1-(3,4-Dimethoxyphenyl)-3-(α,α,α-trifluor -p-tolyl)-2-propen-1-on auf einmal zu. Die Reaktionslösung wird 2 Stunden bei Rückflusstemperatur gehalten, unter vermindertem Druck eingedampft und mit 50 ml Wasser versetzt. Man extrahiert dreimal mit Methylenchlorid. Die Extrakte werden mit Wasser gewaschen, über wasserfreiem Natriumsulfat getrocknet und unter vermindertem Druck eingedampft. Der ölige Rückstand wird an Kieselgel mit Aethylacetat als Laufmittel chromatographisch gereinigt. Auf diese Weise erhält man mit der 1. Fraktion 1,85 g 4-[(all-E)-3-(3,4-Dimethoxyphenyl)-5-(α,α,α-trifluor-p-tolyl) -2,4-pentadienoyl]-mor pholin als weissen Schaum. Die 2. Fraktion liefert 0,52 g 4-[(2-Z,4-E)-3-(3,4-Dimethoxyphenyl) -5-(α,α,α-trifluor-p-tolyl) -2,4-pentadienoyl]-morpholin als weissen Schaum;
Massenspektrum (1. und 2. Isomer) m/e: $M^+$ 447 (60), 428 (2), 361 (100);
$^1$H-NMR ($CDCl_3$, 250 MHz): 1. Isomer 3,89 ppm ( s, $OCH_3$), 3,94 ppm (s, $OCH_3$), 6,08 ppm (s, = CH-CO-), 6,62 ppm (d, 16 Hz, -CH = CH-), 7,88 ppm (d, 16 Hz, -CH = CH-); 2. Isomer 3,86 ppm (s, $OCH_3$), 3,94 ppm (s, $OCH_3$), 6,19 ppm (s, = CH-CO-), 6,56 ppm (d, 16 Hz, -CH = CH-), 7,05 ppm (d, 16 Hz, -CH = CH-).

In analoger Weise erhält man aus:

- (E)-3-(4-Cyanophenyl)-1-(3,4-dimethoxyphenyl)-2-propen-1-on und Diäthylphosphonoessigsäuremorpholid das 4-[(all-E)-5-(4-Cyanophenyl)-3-(3,4-dimethoxyphenyl) -2,4-pentadienoyl]-morpholin als gelblichen Schaum (1. Isomer) und das 4-[(2-Z,4-E)-5-(4-Cyanophenyl)-3-(3,4-dimethoxyphenyl)-2,4-pentadienoyl]-morpholin als gelbes Harz (2. Isomer); Massenspektrum (1. und 2. Isomer) m/e: $M^+$ 404 (64), 318 (100);
$^1$H-NMR ($CDCl_3$, 250 MHz): 1. Isomer 3,89 ppm (s, $OCH_3$), 3,94 ppm (s, $OCH_3$), 6,11 ppm (s, = CH-CO-), 6,59 ppm (d, 16 Hz, -CH = CH-), 7,94 ppm (d, 16 Hz, -CH = CH-);
2. Isomer 3,86 ppm (s, $OCH_3$), 3,94 ppm (s, $OCH_3$), 6,21 ppm (s, = CH-CO-), 6,53 ppm (d, 16 Hz, -CH = CH-), 7,07 ppm (d, 16 Hz, -CH = CH-);
- (E)-3-(4-Biphenylyl)-3′,4′-dimethoxyacrylophenon und Diäthylphosphonoessigsäuremorpholid das 4-[(all-E)-5-(4-Biphenylyl)-3-(3,4-dimethoxyphenyl) -2,4-pentadienoyl]-morpholin als gelbliches Harz, Smp. 119-129 °C;
$^1$H-NMR ($CDCl_3$, 400 MHz): 3,90 ppm (s, $OCH_3$), 3,94 ppm (s, $OCH_3$), 6,00 ppm (s, = CH-CO-), 6,65 ppm (d, 16 Hz, -CH = CH-), 7,81 ppm (d, 16 Hz, -CH = CH-); und das
4-[(2-Z,4-E)-5-(4-Biphenylyl)-3-(3,4-dimethoxyphenyl)-2,4 pentadienoyl]-morpholin als gelben Schaum, Smp. 60-65 °C. Massenspektrum m/e: $M^+$ 455 (97), 369 (100), 341 (43), 252 (20), 165 (45);
$^1$H-NMR ($CDCl_3$, 400 MHz): 3,87 ppm (s, $OCH_3$), 3,94 ppm (s, $OCH_3$), 6,15 ppm (s, = CHCO-), 6,59 ppm (d, 16 Hz, -CH = CH-), 7,02 ppm (d, 16 Hz, -CH = CH-);
- (E)-3-(m-Phenoxyphenyl)-3′,4′-dimethoxyacrylophenon und Diäthylphosphonoessigsäuremorpholid das 4-[(all-E)-3-(3,4-Dimethoxyphenyl)-5-(m-phenoxyphenyl) -2,4-pentadienoyl]-morpholin als gelbliches Harz,

Massenspektrum m/e: $M^+$ 471 (100), 385 (86), 356 (57), 264 (42), 165 (35), 77 (80);
$^1$H-NMR ($CDCl_3$, 400 MHz): 3,88 ppm (s, $OCH_3$), 3,92 ppm (s, $OCH_3$), 5,99 ppm (s, =CHCO-), 6,55 ppm (d, 16 Hz, -CH=CH-), 7,66 ppm (d, 16 Hz, -CH=CH-);
- (E)-3-[m-(1,2-Dichlorvinyloxy)-phenyl] -3',4'-dimethoxy- acrylophenon und Diäthylphosphonoessigsäuremorpholid das 4-[(2-E/Z, 4-E)-5-[m-(1,2-Dichlorvinyloxy)-phenyl]-3-(3,4-dimethoxyphenyl)-2,4-pentadienoyl]-morpholin als gelbliches Harz,
Massenspektrum m/e: $M^+$ 489 (95), 403 (100), 165 (50);
$^1$H-NMR ($CDCl_3$, 400 MHz): 3,89 ppm (s, $OCH_3$), 3,94 ppm (s, $OCH_3$), 5,96 ppm (s, =CHCl), 6,03 ppm (s, =CHCO-), 6,58 ppm (d, 16 Hz, -CH=CH-), 7,73 ppm (d, 16 Hz, -CH=CH-);
- (E)-3-(p-Phenoxyphenyl)-3',4'-dimethoxyacrylophenon und Diäthylphosphonoessigsäuremorpholid das 4-[-(all-E)-3-(3,4-Dimethoxyphenyl)-5-(p-phenoxyphenyl)-2,4-pentadienoyl]-morpholin, Smp. 137-140° C;
$^1$H-NMR ($CDCl_3$, 400 MHz): 3,88 ppm (s, $OCH_3$), 3,93 ppm (s, $OCH_3$), 5,96 ppm (s, =CHCO-), 6,85 ppm (d, 16 Hz, -CH=CH-), 7,68 ppm (d, 16 Hz, -CH=CH-);
Massenspektrum m/e: $M^+$ 471 (82), 385 (100), 357 (42);
- (E)-3-[p-[(α,α,α-Trifluor-p-tolyl)oxy]phenyl]-3',4'-dimethoxyacrylophenon und Diäthylphosphonoessigsäuremorpholid das 4-[(2 E/Z,4E)-3-(3,4-Dimethoxyphenyl)-5-[p-[(α,α,α-trifluor-p-tolyl)-oxy]phenyl]-2,4-pentadienoyl]-morpholin als Harz,
Massenspektrum m/e: $M^+$ 539 (77), 453 (100).

## Beispiel 2

Zu einer eisgekühlten Lösung von 653 mg Morpholin in 10 ml Pyridin tropft man langsam unter Rühren und Feuchtigkeitsausschluss 0,22 ml Phosphortrichlorid. Nach halbstündigem Rühren bei Raumtemperatur werden 1,78 g (2-E/Z,4E)-3-(3,4-Dimethoxyphenyl) -5-(4-methylthiophenyl)-2,4-pentadiensäure auf einmal zugegeben. Man rührt 16 Stunden bei 40° C und versetzt mit Eis und 25%-iger Salzsäure bis zur stark sauren Reaktion. Der ölige Niederschlag wird zweimal mit je 50 ml Aethylacetat extrahiert, und die vereinigten organischen Phasen werden mit Wasser gewaschen und über wasserfreiem Natriumsulfat getrocknet. Nach dem Eindampfen unter vermindertem Druck bleiben 2,2 g grünes Oel zurück, das sich beim Trocknen bei 0,1 mmHg Druck zu einem gelblichen Schaum bestehend aus 4-[(2-E/Z,4-E)-3-(3,4-Dimethoxyphenyl)-5-(4-methylthiophenyl) -2,4-pentadienoyl]-morpholin verfestigt.
$^1$H-NMR ($CDCl_3$, 250 MHz): 2,48 ppm (s, S-$CH_3$), 3,88 ppm (s, O-$CH_3$), 3,93 ppm (s, $OCH_3$), 5,97 ppm (s, -CHCO-), 6,55 ppm (d, 16 Hz, -CH=CH-), 7,74 ppm (d, 16 Hz, -CH=CH-).

In analoger Weise erhält man aus:
- (2-E/Z,4-E)-3-(3,4-Dimethoxyphenyl)-5-(α,α,α-trifluor-m-tolyl)-2,4-pentadiensäure und Morpholin das (2-E/Z,4-E)-4-[3-(3,4-Dimethoxyphenyl)-5-(α,α,α-tri- fluor-m-tolyl)-2,4-pentadienoyl]-morpholin als grünlichen Schaum;
Massenspektrum m/e: $M^+$ 447 (60) 428 (1), 361 (100);
$^1$H-NMR ($CDCl_3$, 250 MHz): 3,89 ppm (s, $OCH_3$), 3,94 ppm (s, $OCH_3$), 6,06 ppm (s, =CHCO-), 6,62 ppm (d, 16 Hz, -CH=CH-), 7.84 ppm (d, 16 Hz, -CH=CH-);
- (2-E/Z,4-E)-5-(4-Cyanophenyl)-3-(3,4-dimethoxyphenyl)-2,4-pentadiensäure und Morpholin das 4-[(all-E)-5-(4-Cyanophenyl)-3-(3,4-dimethoxyphenyl) -2,4-pentadienoyl]-morpholin als gelblichen Schaum;
Massenspektrum m/e: $M^+$ 404 (64), 318 (100);
$^1$H-NMR ($CDCl_3$, 250 MHz): 3,89 ppm (s, $OCH_3$), 3,94 ppm (s, $OCH_3$), 6,11 ppm (s, =CH-CO-), 6,59 ppm (d, 16 Hz, -CH=CH-), 7,94 ppm (d, 16 Hz, -CH=CH-);
- (all-E)-3-(3,4-Dimethoxyphenyl) -5-(α,α,α-trifluor-p-tolyl)-2,4-pentadiensäure und Thiomorpholin das 4-[(all-E)-3-(3,4-Dimethoxyphenyl) -5-(α,α,α-trifluor-p-tolyl)-2,4-pentadienoyl] -thiomorpholin als rosafarbenen Schaum;
Massenspektruk m/e: $M^+$ 463 (64), 444 (6), 361 (100);
$^1$H-NMR ($CDCl_3$, 250 MHz): 3,89 ppm (s, -$OCH_3$, 3,94 ppm (s, -$OCH_3$), 6,07 ppm (s, =CH-CO-), 6,62 ppm (d, 16 Hz, -CH=CH-), 7,79 ppm (d, 16 Hz, -CH=CH-);
- (2-E/Z,4-E)-3-(3,4-Dimethoxyphenyl)-2-methyl-5-(α,α,α-trifluor-p-tolyl)-2,4-pentadiensäure und Morpholin das 4-[(2-E/Z,4-E)-3-(3,4-Dimethoxyphenyl)-2-methyl-5-(α,α,α-trifluor-p-tolyl)-2,4-pentadienoyl]-morpholin als gelbes Oel;
Massenspektrum m/e: $M^+$ 461 (100), 375 (89);
$^1$H-NMR ($CDCl_3$, 250 MHz): 1,83 ppm (s, =-CH)$_3$), 3,88 ppm (s, O-$CH_3$), 3,95 ppm (s, O-$CH_3$), 6,13 ppm (d, 15 Hz, -CH=CH-), 7,06 ppm (d, 15 Hz, -CH=CH-);

- (2-E/Z,4-E)-3-(3,4-Dimethoxyphenyl)-5-(α,α,α,4-tetrafluor-m-tolyl)-2,4-pentadiensäure und Morpholin das 4-(2-E/Z,4-E)-3-(3,4-Dimethoxyphenyl)-5-(α,α,α,4-tetrafluor-m-tolyl)-2,4-pentadienoyl]-morpholin als gelblichen Schaum;
Massenspektrum m/e: $M^+$ 465 (55), 379 (100);
$^1$H-NMR ($CDCl_3$, 250 MHz): 3,89 ppm (s, O-$CH_3$), 3,94 ppm (s, O-$CH_3$), 5,98 ppm (s, =CHCO-): Isomer I (40%), 6,06 ppm (s, -CHCO-): Isomer II (60%);
- (all-E)-3-(3,4-Dimethoxyphenyl)-5-[p-(1,1,2,2-tetrafluoräthoxy)-phenyl]-2,4-pentadiensäure und Morpholin das 4-{(2-E,4-E)-3-(3,4-Dimethoxyphenyl)-5-[p-(1,1,2,2-tetrafluoräthoxy)-phenyl]-2,4-pentadienoyl}-morpholin, Smp. 112-115° C;
Massenspektrum m/e: $M^+$ 495 (67), 409 (100), 381 (18);
- (all-E)-3-(3,4-Dimethoxyphenyl)-5-[p-(trifluormethoxy)-phenyl]-2,4-pentadiensäure und Morpholin das 4-[3-(3,4-Dimethoxyphenyl)-5-[p-(trifluoromethoxy)-phenyl]-2,4-pentadienoyl]-morpholin als Schaum;
Massenspektrum m/e: $M^+$ 463 (64), 377 (100), 349 (22);
- (all-E)-3-(3,4-Dimethoxyphenyl)-5-[p-(trifluormethoxy)-phenyl]-2,4-pentadiensäure und N-Aethyl-N-methylamin das (all-E)-N-Aethyl-N-methyl-3-(3,4-dimethoxyphenyl)-5-[p-(trifluormethoxy)-phenyl]-2,4-pentadiensäureamid als Harz;
Massenspektrum m/e: $M^+$ 435 (83), 377 (100), 349 (18);
$^1$H-NMR ($CDCl_3$, 400 MHz): 1,21 ppm (t, 7.5 Hz, -$CH_2CH_3$), 3,06 ppm (s, N-$CH_3$), 3,50 ppm (q, -$CH_2CH_3$), 3,88 ppm (s, $OCH_3$), 3,94 ppm (s, $OCH_3$), 6,08 ppm (s, =CH-CO-), 6,54 ppm (d, 16 Hz, -CH=CH-), 7,88 ppm (d, 16 Hz, -CH=CH-);
- (all-E)-3-(3,4-Dimethoxyphenyl)-5-[4-chlor-3-(trifluormethyl)-phenyl]-2,4-pentadiensäure und Morpholin das 4-[(all-E)-5-[4-Chlor-3-(trifluormethyl)-phenyl]-3-(3,4- dimethoxyphenyl)-2,4-pentadienoyl]-morpholin als Harz;
Massenspektrum m/e: $M^+$ 481 (70), 395 (100);
$^1$H-NMR ($CDCl_3$, 400 MHz): 3,89 ppm (s, $OCH_3$), 3,94 ppm (s, $OCH_3$), 6,08 ppm (s, =CH-CO-), 6,55 ppm (d, 16 Hz, -CH=CH-), 7,86 ppm (d, 16 Hz, -CH=CH-);
- (all-E)-3-(3-Fluor-4-methoxyphenyl)-5-(α,α,α-trifluor-p-tolyl)-2,4-pentadiensäure und Morpholin das 4-[(all-E)-3-(3-Fluor-4-methoxyphenyl)-5-(α,α,α-trifluor-p-tolyl)-2,4-pentadienoyl]-morpholin als Harz;
Massenspektrum m/e: $M^+$ 435 (74), 416 (6), 349 (100);
- (all-E)-3-(4-Methoxyphenyl)-5-(α,α,α-trifluor-p-tolyl)-2,4-pentadiensäure und Morpholin das 4-[(all-E)-3-(4-Methoxyphenyl)-5-(α,α,α-trifluor-p-tolyl)-2,4-pentadienoyl]-morpholin als Harz;
Massenspektrum m/e: $M^+$ 417 (56), 398 (3), 331 (100), 303 (12);
$^1$N-NMR ($CDCl_3$, 400 MHz): 3,87 ppm (s, $OCH_3$), 6,05 ppm (s, =CH-CO-), 6,59 ppm (d, 16 Hz, -CH=CH-), 7.91 ppm (d, 16 Hz, -CH=CH-);
- (all-E)-3-(3,4-Dimethoxyphenyl)-5-(α,α,α-trifluor-m-tolyl)-2,4-pentadiensäure und N-Aethyl-N-methylamin das (all-E)-N-Aethyl-N-methyl-3-(3,4-dimethoxyphenyl)-5-(α,α,α-trifluor-m-tolyl)-2,4-pentadiensäureamid, Schmelzpunkt 88-93° C;
Massenspektrum m/e: $M^+$ 419 (84), 400 (3), 361 (100);
- 3-(3,4-Dimethoxyphenyl)5-(α,α,α-trifluor-p-tolyl)-2,4-pentadiensäure und N-Aethyl-N-methylamin das (all-E)-N-Aethyl-N-methyl-3-(3,4-dimethoxyphenyl)-5-(α,α,α-trifluor-p-tolyl)-2,4-pentadiensäureamid als Harz;
Massenspektrum m/e: $M^+$ 419 (90), 400 (5), 361 (100);
$^1$H-NMR ($CDCl_3$, 400 MHz): 1,21 ppm (t, -$CH_2CH_3$), 3,07 ppm (s, -$NCH_3$), 3,51 ppm (q, -$CH_2CH_3$), 3,89 ppm (s, $OCH_3$), 3,94 ppm (s, $OCH_3$), 6,12 ppm (s, =CHCO-), 6,59 ppm (d, 16 Hz, -CH=CH-), 7,99 ppm (d, 16 Hz, -CH=CH-); und
das (2-Z,4-E)-N-Aethyl-N-methyl-3-(3,4-dimethoxyphenyl)-5-(α,α,α-trifluor-p-tolyl)-2,4-pentadiensäureamid als Harz;
$^1$H=NMR ($CDCl_3$, 400 MHz): 0,91 ppm und 0,98 ppm je (t, -$CH_2CH_3$), 2,74 ppm und 2,80 ppm je (s, -$NCH_3$), 3,25 ppm und 3,29 ppm je (q, -$CH_2CH_3$), 3,86 ppm (s, $OCH_3$), 3,92 ppm (s, $OCH_3$), 6,25 ppm und 6,26 ppm je (s, =CH-CO-), 6,51 ppm und 6,52 ppm je (d, 16 Hz, CH=CH), 7,06 ppm (d, 16 Hz, -CH=CH):
- (all-E)-3-(3,4-Dimethoxyphenyl)-5-(α,α,α-trifluor-p-tolyl)-2,4-pentadiensäure und N-Isobutyl-N-methylamin das (all-E)-N-Isobutyl-N-methyl-3-(3,4-dimethoxyphenyl)-5-(α,α,α-trifluor-p-tolyl)-2,4-pentadiensäureamid als Harz;
Massenspektrum m/e: $M^+$ 447 (69), 428 (5), 404 (5), 361 (100);
- (all-E)-3-(3,4-Dimethoxyphenyl)-5-(α,α,α-trifluor-p-tolyl)-2,4-pentadiensäure und N-Aethyl-N-butylamin das (all-E)-N-Aethyl-N-butyl-3-(3,4-dimethoxyphenyl)-5-(α,α,α-trifluor-p-tolyl)-2,4-pentadiensäureamid als Sirup;
Massenspektrum m/e: $M^+$ 461 (78), 442 (6), 361 (100), 334 (38), 302 (24), 165 (35), 100 (55);
- 3-(3,4-Dimethoxyphenyl)-2-methyl-5-(α,α,α-trifluor-p-tolyl)-2,4-pentadiensäure und N-Aethyl-N-methylamin das N-Aethyl-N-methyl-2-methyl-3-(3,4-dimethoxyphenyl)-5-(α,α,α-trifluor-p-tolyl)-2,4-pentadiensäureamid als Harz;

Massenspektrum m/e: $M^+$ 433 (100), 418 (12), 414 (6), 375 (86), 347 (43), 165 (46);

- (all-E)-3-(3,4-Dimethoxyphenyl)-5-[4-chlor-3-(trifluormethyl)-phenyl]-2,4-pentadiensäure und N-Aethyl-N-methylamin das (all-E)-N-Aethyl-N-methyl-5-[4-chlor-3-(trifluormethyl)-phenyl]-3-(3,4-dimethoxyphenyl)-2,4-pentadiensäureamid, Smp. 115-119 °C; Massenspektrum m/e: $M^+$ 453 (84), 395 (100):
- (all-E)-3-(3,4-Dimethoxyphenyl)-5-[4-chlor-3-(trifluormethyl)-phenyl]-2,4-pentadiensäure und N,N-Dimethylamin das (all-E)-N,N-Dimethyl-5-[4-chlor-3-(trifluormethyl)-phenyl]-3-(3,4-dimethoxyphenyl)-2,4-pentadiensäureamid,Smp. 100-103 °C; Massenspektrum m/e: $M^+$ 439 (64), 395 (100);
- (all-E)-3-(3,4-Dimethoxyphenyl)-5-{m-[(α,α,α-trifluor-m-tolyl)oxy]-phenyl{-2,4-pentadiensäure und Morpholin das 4-[(all-E)-3-(3,4-Dimethoxyphenyl)-5-{m-[(α,α,α-trifluor-m-tolyl)oxy]-phenyl}-2,4-pentadienoyl]-morpholin als Schaum;

Massenspektrum m/e: $M^+$ 539 (100), 453 (78), 425 (35), 264 (29);

$^1$H-NMR ($CDCl_3$, 400 MHz): 3,88 ppm (s, $OC\underline{H}_3$), 3,93 ppm (s, $OC\underline{H}_3$), 6,02 ppm (s, $=C\underline{H}CO-$), 6,56 ppm (d, 16 Hz, $-C\underline{H}=CH-$), 7,73 ppm (d, 16 Hz, $-CH=C\underline{H}-$);

- 3-(3,4-Dimethoxyphenyl)-5-(4-fluor-3-phenoxyphenyl)-2,4-pentadiensäure und Morpholin das 4-[(all-E)-3-,4-Dimethoxyphenyl)-5-(4-fluor-3-phenoxyphenyl)-2,4-pentadienoyl]-morpholin als Harz;

Massenspektrum m/e: $M^+$ 490 (92), 403 (100), 375 (40);

$^1$H-NMR ($CDCl_3$, 400 MHz): 3,87 ppm (s, $OC\underline{H}$), 3,92 ppm (s, $OC\underline{H}_3$), 5,99 (s, $=C\underline{H}CO-$), 6,48 ppm (d, 16 Hz. $-C\underline{H}=CH-$), 7,62 ppm (d, 16 Hz, $-CH=C\underline{H}-$);

und das 4-[(2-Z,4-E)-3-(3,4-Dimethoxyphenyl)-5-(4-fluor-3-phenoxy-phenyl)-2,4-pentadienoyl]-morpholin als Harz;

$^1$H-NMR ($CDCl_3$, 400 MHz): 3,84 ppm (s, $OC\underline{H}_3$), 3,92 ppm (s, $OC\underline{H}_3$), 6,09 ppm (s, $=C\underline{H}CO-$), 6,42 ppm (d, 16 Hz, $-C\underline{H}=CH-$), 6,82 ppm (d, 16 Hz, $-CH=C\underline{H}-$);

- (all-E)-3-(3,4-Dimethoxyphenyl)-5-[m-(3,5-dichlorphenoxy)-phenyl]-2,4-pentadiensäure und Morpholin das 4-{(all-E)-5-[m-(3,5-Dichlorphenoxy)-phenyl]-3-(3,4-dimethoxyphenyl)-2,4-pentadienoyl}-morpholin als Schaum;

Massenspektrum m/e: $M^+$ 539 (100), 453 (45), 425 (32);

$^1$H-NMR ($CDCl_3$, 400 MHz): 3,89 ppm (s, $OCH_3$), 3,93 ppm (s, $OC\underline{H}_3$), 6,02 ppm (s, $=C\underline{H}-CO-$), 6,56 ppm (d, 16 Hz, $-C\underline{H}=CH-$), 7,75 ppm (d, 16 Hz. $-CH=C\underline{H}-$).

## <u>II. Herstellung der Ausgangsmaterialien der Formeln II und IV:</u>

### Beispiel 3

Zu einer Lösung von 2 ml 15%-iger Kalilauge in 10 ml Methanol tropft man eine Lösung von 36 g 3,4-Dimethoxyacetophenon und 36 g 4-Trifluormethylbenzaldehyd in 200 ml Methanol so zu, dass die Temperatur 30 °C nicht übersteigt. Das Reaktionsgemisch wird 72 Stunden bei Raumtemperatur weitergerührt, danach der ausgefallene Niederschlag abgesaugt und aus Aethanol umkristallisiert. Dabei erhält man 53 g (E-1-(3,4-Dimethoxyphenyl)-3-(α,α,α-trifluor-p-tolyl)-2-propen-1-on als weisse Kristalle, Smp. 83 °C; Massenspektrum m/e: $M^+$ 336 (100), 199 (14), 165 (84);

$^1$H-NMR ($CDCl_3$, 250 MHz): 3,98 ppm (s, 6H, $OC\underline{H}_3$), 6,95 ppm (d, 8 Hz, $-C\underline{H}=CH-$).

In analoger Weise erhält man aus:

- 3,4-Dimethoxyactophenon und 4-Cyanobenzaldehyd das (E)-3-(4-Cyanophenyl)-1-(3,4-dimethoxyphenyl) -2-propen-1-on als hellgelbe Kristalle, Smp. 169-171 °C;

Massenspektrum m/e: $M^+$ 293 (100), 265 (35), 262 (32), 165 (73);

$^1$H-NMR ($CDCl_3$, 250 MHz): 3,98 pm (s, 6H, $-OCH_3$), 6,95 ppm (d, 8 Hz, $-C\underline{H}=CH-$);

- 3,4-Dimethoxyacetophenon und 4-Phenylbenzaldehyd das (E)-3-(4-Biphenylyl)-3′,4′-dimethoxyacrylophenon, Smp. 135-137 °C; Massenspektrum m/e: $M^+$ 344 (100), 329 (12), 313 (22), 267 (16), 165 (28);
- 3,4-Dimethoxyacetophenon und m-(1,2-Dichlorvinyloxy)-benzaldehyd das (E)-3-[m-(1,2-Dichlorvinyloxy)-phenyl] -3′,4′-dimethoxyacrylophenon als gelbliches Harz, Massenspektrum m/e: $M^+$ 378 (81), 267 (17), 165 (100).
- 3,4-Dimethoxyacetophenon und m-Phenoxybenzaldehyd das (E)-3-(m-Phenoxyphenyl)-3′,4′-dimethoxyacrylophenon als Harz;

Massenspektrum m/e: $M^+$ 360 (100), 329 (27), 267 (35), 165 (48);

- 3,4-Dimethoxyacetophenon und p-Phenoxybenzaldehyd das (E)-3-(p-Phenoxyphenyl)-3',4'-dimethoxyacrylophenon, Smp. 120-123 °C;
- 3,4-Dimethoxyacetophenon und p-[(α,α,α-Trifluor-p-tolyl)oxy]-benzaldehyd das (E)-3-{p-[(α,α,α-trifluor-p-tolyl)oxy]-phenyl}-3',4'-dimethoxyacrylophenon, Smp. 120-122 °C.

## Beispiel 4

Zu einer eisgekühlten Lösung von 2,47 g Kalium-tert.-butylat in 10 ml Dimethylformamid tropft man während 10 Minuten unter Rühren eine Lösung von 5,00 g (E/Z)-3,4-Dimethoxy-β-methyl-zimtsäure-äthylester und 3,20 g 4-Methylthiobenzaldehyd in 15 ml Dimethylformamid. Danach wird eine Stunde bei Raumtemperatur weitergerührt, mit 100 ml Wasser versetzt und zweimal mit je 50 ml Diäthyläther extrahiert. Zur wässrigen Phase gibt man 7,5 ml 4N Salzsäure. Der entstandene Niederschlag wird abgesaugt, mit Wasser gewaschen und getrocknet. Nach Umkristallisation aus Toluol/Aethylacetat erhält man 5,5 g (2-E/Z,4-E)-3-(3,4-Dimethoxyphenyl)-5-(4-methylthiophenyl) -2,4-pentadiensäure als gelbe Kristalle, Smp. 173-179 °C;
Massenspektrum m/e: $M^+$ 356 (45), 311 (61), 264 (100);
$^1$H-NMR (DMSO-$d_6$, 250 MHz): 2,49 ppm (s, $SCH_3$), 3,78 ppm (s, $OCH_3$), 3,81 ppm (s, $OCH_3$), 5,74 ppm (s, =CHCO-), 6,63 ppm (d, 16 Hz, -CH=CH-), 8,35 ppm (d, 16 Hz, -CH=CH-), 12,3 ppm (s (breit), -COOH).

In analoger Weise erhält man aus:
- (E/Z)-3,4-Dimethoxy-β-methyl-zimtsäure-äthylester und 3-Trifluormethylbenzaldehyd die (2-E/Z,4-E)-3-(3,4-Di-methoxyphenyl) -5-(α,α,α-trifluor-m-tolyl)-2,4-pentadiensäure als gelbe Kirstalle, Smp. 150-153 °C;
Massenspektrum m/e: $M^+$ 378 (82), 333 (100), 302 (20);
$^1$H-NMR (DMSO-$d_6$, 250 MHz): 3,79 ppm (s, $OCH_3$), 3,81 ppm (s, $OCH_3$), 5,85 ppm (s, =CHCO-), 6,79 ppm (d, 16 Hz, -CH=CH-), 8,42 ppm (d, 16 Hz, -CH=CH-), 12,5 ppm (s (breit), COOH).
- (E/Z)-3,4-Dimethoxy-β-methyl-zimtsäure -äthylester und 4-Cyanobenzaldehyd die (2-E/Z,4-E)-5-(4-Cyanophenyl)-3-(3,4-dimethoxyphenyl)-2,4-pentadiensäure als gelbe Kristalle, Smp. 212-213 °C (Zers.);
Massenspektrum m/e: $M^+$ 335 (49), 290 (100);
$^1$H-NMR (DMSO-$d_6$, 250 MHz): 3,78 ppm (s, $OCH_3$), 3,81 ppm (s, $OCH_3$), 5,88 pm (s, =CH-CO-), 6,75 ppm (d, 16 Hz, -CH=CH-), 8,47 ppm (d, 16 Hz, -CH=CH-), 12,5 ppm (s (breit), COOH);
- (E/Z)-3,4-Dimethoxy-β-methyl-zimtsäure-äthylester und 4-Trifluormethylbenzaldehyd die (all-E)-3-(3,4-Dimethoxyphenyl)-5-(α,α,α-trifluor -p-tolyl)-2,4-pentadiensäure als gelbe Kristalle, Smp. 201-203 °C;
- (E/Z)-3,4-Dimethoxy-β-methyl-zimtsäure-äthylester und α,α,α-Tetrafluor-m-tolylaldehyd die (2-E/Z,4-E)-3-(3,4-Dimethoxyphenyl)-5-(α,α,α,4- tetrafluor-m-tolyl)-2,4-pentadiensäure als gelbe Kristalle, Smp. 150-153 °C;
Massenspektrum m/e: $M^+$ 396 (91), 377 (25), 351 (100);
- (E/Z)-3,4-Dimethoxy-α,β-dimethyl-zimtsäure-äthylester und 4-Trifluormethylbenzaldehyd die (2-E/Z,4-E)-3-(3,4-Dimethoxyphenyl)-2-methyl-5-(α,α,α-trifluor-p-tolyl)-2,4-pentadiensäure als gelben Schaum;
Massenspektrum m/e: $M^+$ 392 (71), 347 (65);
- (E/Z)-3,4-Dimethoxy-β-methyl-zimtsäure-äthylester und p-(1,1,2,2-Tetrafluoräthoxy)-benzaldehyd die (all-E)-3-(3,4-Dimethoxyphenyl)-5-[p-(1,1,2,2-tetrafluoräthoxy)-phenyl]-2,4-pentadiensäure, Smp. 179-182 °C;
- (E/Z)-3,4-Dimethoxy-β-methyl-zimtsäure-äthylester und p-(Trifluormethoxy)-benzaldehyd die (all-E)-3-(3,4-Dimethoxyphenyl)-5-[p-(trifluormethoxy)-phenyl]-2,4-pentadiensäure, Smp. 188-190 °C;
- (E/Z)-3,4-Dimethoxy-β-methyl-zimtsäure-äthylester und 4-Chlor-3-(trifluormethyl)-benzaldehyd die (all-E)-3-(3,4-Dimethoxyphenyl)-5-[4-chlor-3-(trifluormethyl)-phenyl]-2,4-pentadiensäure, Smp. 175 °C (Zers.); - (E/Z)-3,4-Dimethoxy-β-methyl-zimtsäure-äthylester und m-[(α,α,α-Trifluor-m-tolyl)oxy]benzaldehyd die (all-E)-3-(3,4-Dimethoxyphenyl)-5{m-[(α,α,α-trifluor-m-tolyl}oxy]-phenyl}-2,4-pentadiensäure als Harz;
$^1$H-NMR ($CDCl_3$, 400 MHz): 3,89 ppm (s, $OCH_3$), 3,93 ppm (s, $OCH_3$), 5,89 ppm (s, =CHCO-), 6,69 ppm (d, 16 Hz, -CH=CH-), 8,40 ppm (d, 16 Hz, -CH-CH-);
- (E/Z)-3,4-Dimethoxy-β-methyl-zimtsäure-äthylester und m-((3,5-Dichlorphenoxy)-benzaldehyd die (all-E)-3-(3,4-Dimethoxyphenyl)-5-[m-(3,5-dichlorphenoxy)-phenyl]-2,4-pentadiensäure als Harz;
$^1$H-NMR ($CDCl_3$, 400 MHz): 3,90 ppm (s, $OCH_3$), 3,94 ppm (s, $OCH_3$), 5,80 ppm (s, =CHCO-) 6,68 ppm (d, 16 Hz, -CH=CH-), 8,41 ppm (d, 16 Hz, -CH=CH-);
- (E/Z)-3,4-Dimethoxy-β-methyl-zimtsäure-äthylester und 4-Fluor-3-phenoxybenzaldehyd die 3-(3,4-Dimethoxyphenyl)-5-(4-fluor-3-phenoxyphenyl)-2,4-pentadiensäure, Smp. 116-120 °C;
- (E/Z)-3-Fluor-4-methoxy-β-methyl-zimtsäure-äthylester und 4-Trifluormethylbenzaldehyd die (all-E)-3-(3-Fluor-4-methoxyphenyl)-5-(α,α,α-trifluor-p-tolyl)-2,4-pentadiensäure, Smp. 160-164 °C;

- (E/Z)-4-Methoxy-β-methyl-zimtsäure-äthylester und 4-Trifluormethylbenzaldehyd die (all-E)-3-(4-Methoxyphenyl)-5-(α,α,α-trifluor-p-tolyl)-2,4-pentadiensäure, Smp. 202-203 °C;
- (E/Z)-3,4-Dimethoxy-α,β-dimethyl-zimtsäure-äthylester und 4-Trifluormethylbenzaldehyd die 3-(3,4-Dimethoxyphenyl)-2-methyl-5-(α,α,α-trifluor-p-tolyl)-2,4-pentadiensäure als Harz;
Massenspektrum m/e: $M^+$ 392 (100), 347 (60).

## Beispiel 5

Unter Rühren und Feuchtigkeitsausschluss werden zu einer Suspension von 8,0 g Kalium-tert.butylat in 250 ml 1,2-Dimethoxyäthan 16,0 g Phosphonoessigsäure-triäthylester getropft. Das Reaktionsgemisch wird 2 Stunden bei 50 °C gehalten. 16,0 g (E)-1-(3,4-Dimethoxyphenyl)-3-(α,α,α-trifluor-p-tolyl)-2-propen-1-on werden auf einmal zugegeben. Man erhitzt das Reaktionsgemisch 16 Stunden bei Rückflusstemperatur. Das Lösungsmittel wird unter vermindertem Druck abgedampft und der Rückstand mit Eiswasser versetzt und dreimal mit je 100 ml Methylenchlorid extrahiert. Die vereinigten Extrakte werden mit Wasser gewaschen, über wasserfreiem Natriumsulfat getrocknet und unter vermindertem Druck eingdampft. Nach chromatographischer Reinigung mit Methylenchlorid an Kieselgel erhält man den (2-E/Z,4-E)-3-(3,4-Dimethoxyphenyl) -5-(α,α,α-trifluor-p-tolyl) -2,4-pentadiensäure-äthylester als orangefarbenes Oel.

Eine Lösung von 4,0 g (2-E/Z,4-E)-3-(3,4-Dimethoxyphenyl) -5-(α,α,α-trifluor-p-tolyl) -2,4-pentadiensäure-äthylester in 30 ml Methanol wird mit 11 ml 1N Natronlauge versetzt und das Reaktionsgemisch 3 Stunden bei Rückflusstemperatur gerührt. Man verdünnt mit 50 ml Wasser und säuert mit 2N Salzsäure an. Der entstandene Niederschlag wird abgenutscht, mit Wasser gewaschen und getrocknet. Man erhält 3,4 g (2-E,4-E)-3-(3,4-Dimethoxyphenyl)-5-(α,α,α-trifluor-p-tolyl)-2,4-pentadiensäure als gelbes Pulver, Smp. 201-203 °C;
Massenspektrum m/e: $M^+$ 378 (48), 359 (8), 333 (100);
$^1$H-NMR (DMSO-$d_6$, 250 MHz): 3,78 ppm (s, $OCH_3$), 3,81 ppm (s, $OCH_3$), 5,86 ppm (s, =C<u>H</u>CO-), 6,75 ppm (d, 16 Hz, -C<u>H</u>=CH-), 7,96 ppm (d, 16 HZ, -CH=C<u>H</u>-), 12,5 ppm (s (breit), COO<u>H</u>).

In analoger Weise erhält man aus:
- 3-Fluor-4-methoxyacetophenon und Phosphonoessigsäuretriäthylester den 3-Fluor-4-methoxy-β-methylzimtsäure-äthylester als teilkristallines Produkt, das gemäss $^1$H-NMR ein E/Z-Isomerengemisch im Verhältnis 4:1 darstellt;
Massenspektrum m/e: $M^+$ 238 (97), 193 (100), 166 (65).

## III. Formulierungsbeispiele:

## Beispiel 6

Ein Spritzpulver hat folgende Zusammensetzung:

| | Gewichtsteile |
|---|---|
| Wirkstoff der Formel I | 25 |
| Hydratisierte Kieselsäure | 25 |
| Natrium-lignosulfonat | 10 |
| Natrium-laurylsulfat | 2 |
| Calciumcarbonat | 38 |
| | 100 |

Der flüssige oder geschmolzene Wirkstoff wird auf die Kieselsäure aufgezogen, die übrigen Komponenten werden zugemischt und das Ganze wird in einer geigneten Mühle feingemahlen.

Beispiel 7

Ein emulgierbares Konzentrat hat folgende Zusammensetzung:

| | g/Liter |
|---|---|
| Wirkstoff der Formel I | 250 |
| Polyarylphenol-(18)äthoxylat (Emulgator) | 300 |
| N-Methyl-2-pyrrolidon (Lösungsmittel) ad | 1 l |

Der Wirkstoff und der Emulgator werden ins Lösungsmittel aufgenommen, wobei eine klare, kältebeständige Lösung entsteht. In Wasser gegossen ergibt sich eine nahezu klare Emulsion, die als Spritzbrühe dient.

**Ansprüche**

1. Verbindungen der allgemeinen Formel

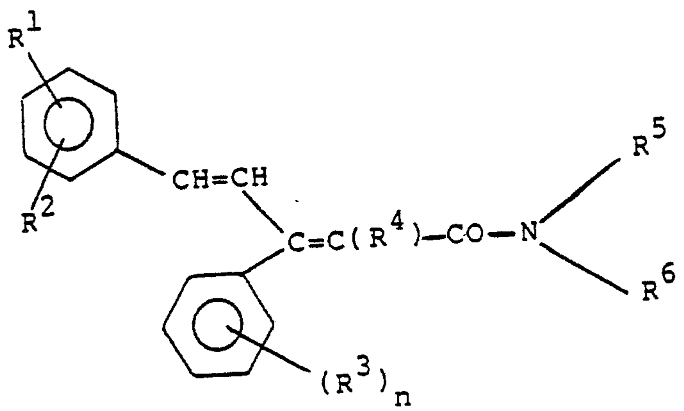

I

worin

$R^1$ $C_{1-6}$-Halogenalkyl, $C_{2-6}$-Halogenalkenyl, $C_{1-6}$-Halogenalkoxy, $C_{2-6}$-Halogenalkenyloxy, $C_{1-6}$-Alkylthio, $C_{1-6}$-Halogenalkylthio, Cyano, gegebenenfalls substituiertes Phenyl oder gegebenenfalls substituiertes Phenoxy,

$R^2$ Wasserstoff oder Halogen,

$R^3$ Halogen, $C_{1-6}$-Alkyl oder $C_{1-6}$-Alkoxy,

$R^4$ Wasserstoff oder $C_{1-6}$-Alkyl,

$R^5$ und $R^6$ unabhängig voneinander $C_{1-4}$-Alkyl oder zusammen mit dem Stickstoffatom, an das sie geknüpft sind, eine Morpholino- oder Thiomorpholinogruppe und

n 1., 2 oder 3 bedeuten.

2. Verbindungen nach Anspruch 1, worin $R^1$ Trifluormethyl oder Trifluormethoxy bedeutet.

3. Verbindungen nach Anspruch 1 oder 2, worin $R^3$ Methoxy bedeutet.

4. Verbindungen nach einem der Ansprüche 1 bis 3, worin sich der bzw. jeder Substituent $R^3$ in der m- oder p-Stellung befindet.

5. Verbindungen nach einem der Ansprüche 1 bis 4, worin $NR^5R^6$ Morpholino oder Thiomorpholino bedeutet.

6. Verbindungen nach einem der Ansprüche 1 bis 4, worin $NR^5R^6$ Aethylmethylamino bedeutet.

7. Eine Verbindung nach Anspruch 1, ausgewählt aus:

4-[3-(3,4-Dimethoxyphenyl)-5($\alpha,\alpha,\alpha$-trifluor-p-tolyl)-2,4-pentadienoyl]-morpholin,

4-[3-(3,4-Dimethoxyphenyl)-5-($\alpha,\alpha,\alpha$-trifluor-p-tolyl)-2,4-pentadienoyl]-thiomorpholin und

N-Aethyl-N-methyl-3-(3,4-dimethoxyphenyl)-5-($\alpha,\alpha,\alpha$-trifluor-p-tolyl)-2,4-pentadiensäureamid.

8. Fungizides Mittel, dadurch gekennzeichnet, dass es eine wirksame Menge mindestens einer Verbindung der allgemeinen Formel

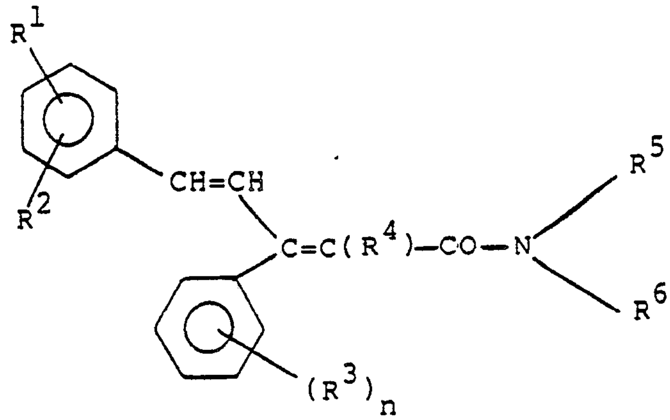

I

worin
$R^1$ $C_{1-6}$-Halogenalkyl, $C_{2-6}$-Halogenalkenyl, $C_{1-6}$-Halogenalkoxy, $C_{2-6}$-Halogenalkenyloxy, $C_{1-6}$-Alkylthio, $C_{1-6}$-Halogenalkylthio, Cyano, gegebenenfalls substituiertes Phenyl oder gegebenenfalls substituiertes Phenoxy,
$R^2$ Wasserstoff oder Halogen,
$R^2$ Halogen, $C_{1-6}$-Alkyl oder $C_{1-6}$-Alkoxy,
$R^4$ Wasserstoff oder $C_{1-6}$-Alkyl,
$R^5$ und $R^6$ unabhängig voneinander $C_{1-4}$-Alkyl oder zusammen mit dem Stickstoffatom, an das sie geknüpft sind, eine Morpholino- oder Thiomorpholinogruppe und
n 1, 2 oder 3 bedeuten,
sowie Formulierungshilfsstoffe enthält.

9. Fungizides Mittel nach Anspruch 8, dadurch gekennzeichnet, dass es eine wirksame Menge mindestens einer aus der Gruppe
4-[3-(3,4-Dimethoxyphenyl)-5-($\alpha,\alpha,\alpha$-trifluor-p-tolyl)-2,4-pentadienoyl]-morpholin,
4-[3-(3,4-Dimethoxyphenyl)-5-($\alpha,\alpha,\alpha$-trifluor-p-tolyl)-2,4-pentadienoyl]-thiomorpholin und
N-Aethyl-N-methyl-3-(3,4-dimethoxyphenyl)-5-($\alpha,\alpha,\alpha$-trifluor-p-tolyl)-2,4-pentadiensäureamid
ausgewählten Verbindung sowie Formulierungshilfsstoffe enthält.

10. Verfahren zur Herstellung von Verbindungen der allgemeinen Formel

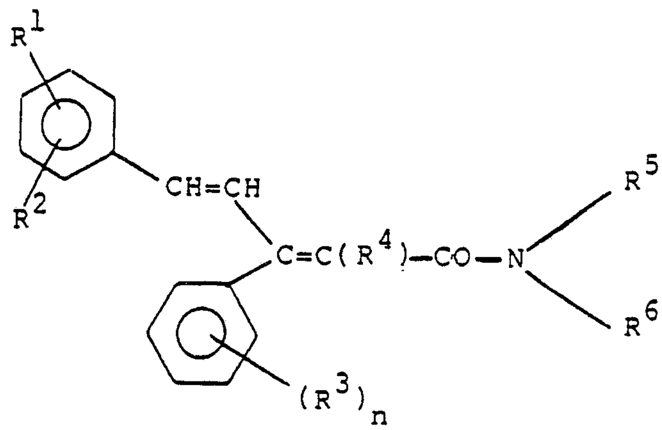

I

worin
$R^1$ $C_{1-6}$-Halogenalkyl, $C_{2-6}$-Halogenalkenyl, $C_{1-6}$-Halogenalkoxy, $C_{2-6}$-Halogenalkenyloxy, $C_{1-6}$-Alkylthio, $C_{1-6}$-Halogenalkylthio, Cyano, gegebenenfalls substituiertes Phenyl oder gegebenenfalls substituiertes Phenoxy,
$R^2$ Wasserstoff oder Halogen,
$R^3$ Halogen, $C_{1-6}$-Alkyl oder $C_{1-6}$-Alkoxy,

$R^4$ Waserstoff oder $C_{1-6}$-Alkyl,
$R^5$ und $R^6$ unabhängig voneinander $C_{1-4}$-Alkyl oder zusammen mit dem Stickstoffatom, an das sie geknüpft sind, eine Morpholino- oder Thiomorpholinogruppe und
n 1, 2 oder 3 bedeuten,
dadurch gekennzeichnet, dass man

a) ein Keton der allgemeinen Formel

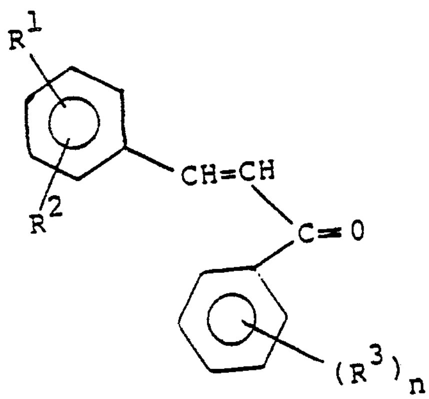

II

worin $R^1$, $R^2$, $R^3$ und n die oben angegebenen Bedeutungen besitzen,
mit einem Phosphonoalkansäureamid der allgemeinen Formel

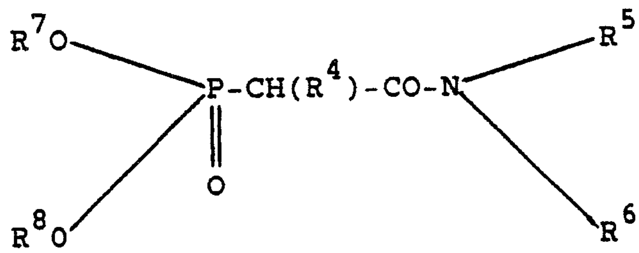

III

worin $R^4$, $R^5$ und $R^6$ die oben angegebenen Bedeutungen besitzen und $R^7$ und $R^8$ unabhängig voneinander eine Niederalkylgruppe oder Arylgruppe bedeuten,
umsetzt, oder

b) eine Acrylsäure der allgemeinen Formel

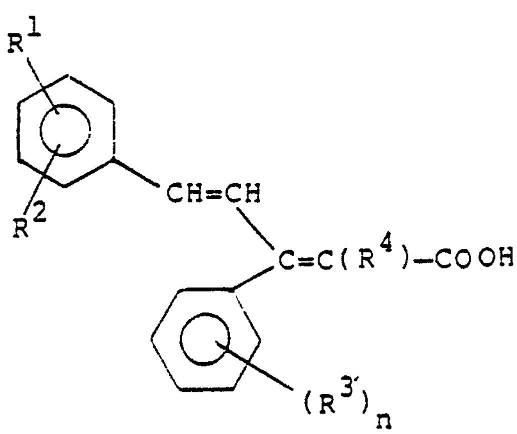

IV

worin $R^1$, $R^2$, $R^3$, $R^4$ und n die oben angegebenen Bedeutungen besitzen,
oder ein reaktionsfähiges Derivat davon mit einem Amin der allgemeinen Formel

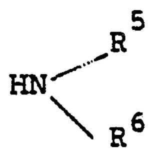

V

worin $R^5$ und $R^6$ die oben angegebenen Bedeutungen besitzen, oder mit einem reaktionsfähigen Derivat davon umsetzt.

11. Verfahren zur Bekämpfung von Fungi in der Landwirtschaft und im Gartenbau, dadurch gekennzeichnet, dass man das zu schützende Gut mit einer wirksamen Menge einer Verbindung gemäss einem der Ansprüche 1 bis 5 und 7 bzw. eines Mittels gemäss Anspruch 8 oder 9 behandelt.

12. Verfahren zur Bekämpfung von Fungi in der Landwirtschaft und im Gartenbau, dadurch gekennzeichnet, dass man das zu schützende Gut mit einer wirksamen Menge einer Verbindung gemäss Anspruch 6 behandelt.

13. Verwendung einer Verbindung gemäss einem der Ansprüche 1 bis 5 und 7 bzw. eines Mittels gemäss Anspruch 8 oder 9 zur Bekämpfung von Fungi in der Landwirtschaft und im Gartenbau.

14. Verwendung einer Verbindung gemäss Anspruch 6 zur Bekämpfung von Fungi in der Landwirtschaft und im Gartenbau.

Patentansprüche für den folgenden Vertragsstaat: ES

1. Fungizides Mittel, dadurch gekennzeichnet, dass es eine wirksame Menge mindestens einer Verbindung der allgemeinen Formel

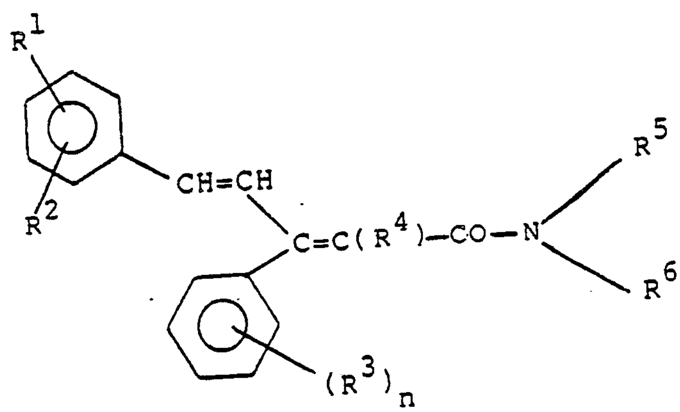

I

worin
$R^1$ $C_{1-6}$-Halogenalkyl, $C_{2-6}$-Halogenalkenyl, $C_{1-6}$-Halogenalkoxy, $C_{2-6}$-Halogenalkenyloxy, $C_{1-6}$-Alkylthio, $C_{1-6}$-Halogenalkylthio, Cyano, gegebenenfalls substituiertes Phenyl oder gegebenenfalls substituiertes Phenoxy,
$R^2$ Wasserstoff oder Halogen,
$R^3$ Halogen, $C_{1-6}$-Alkyl oder $C_{1-6}$-Alkoxy,
$R^4$ Wasserstoff oder $C_{1-6}$-Alkyl,
$R^5$ und $R^6$ unabhängig voneinander $C_{1-4}$-Alkyl oder zusammen mit dem Stickstoffatom, an das sie geknüpft sind, eine Morpholino- oder Thiomorpholinogruppe und
n 1, 2 oder 3 bedeuten,
sowie Formulierungshilfsstoffe enthält.

2. Fungizides Mittel nach Anspruch 1, worin $R^1$ Trifluormethyl oder Trifluormethoxy bedeutet.

3. Fungizides Mittel nach Anspruch 1 oder 2, worin $R^3$ Methoxy bedeutet.

4. Fungizides Mittel nach einem der Ansprüche 1 bis 3, worin sich der bzw. jeder Substituent $R^3$ in der m- oder p-Stellung befindet.

5. Fungizides Mittel nach einem der Ansprüche 1 bis 4, worin $NR^5R^6$ Morpholino oder Thiomorpholino bedeutet.

6. Fungizides Mittel nach einem der Ansprüche 1 bis 4, worin $NR^5R^6$ Aethylmethylamino bedeutet.

7. Fungizides Mittel nach Anspruch 1, dadurch gekennzeichnet, dass es eine wirksame Menge mindestens einer aus der Gruppe:
4-[3-(3,4-Dimethoxyphenyl)-5-(α,α,α-trifluor-p-tolyl)-2,4-pentadienoyl]-morpholin,
4-[3-(3,4-Dimethoxyphenyl)-5-(α,α,α-trifluor-p-tolyl)-2,4-pentadienoyl]-thiomorpholin und
N-Aethyl-N-methyl-3-(3,4-dimethoxyphenyl)-5-(α,α,α-trifluor-p-tolyl)-2,4-pentadiensäureamid
ausgewählten Verbindung sowie Formulierungshilfsstoffe enthält.

8. Verfahren zur Herstellung von Verbindungen der allgemeinen Formel

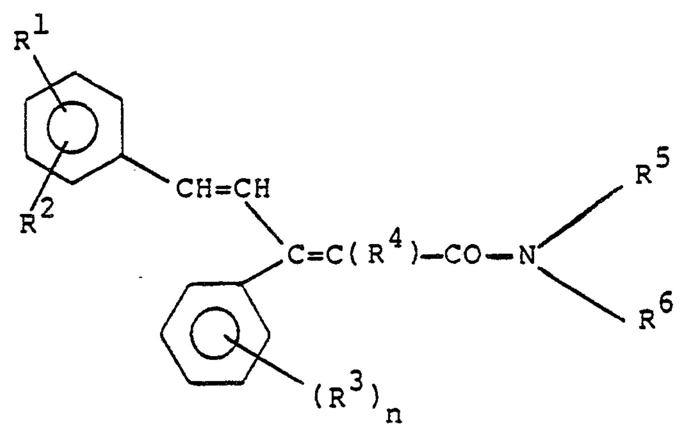

I

worin
$R^1$ $C_{1-6}$-Halogenalkyl, $C_{2-6}$-Halogenalkenyl, $C_{1-6}$-Halogenalkoxy, $C_{2-6}$-Halogenalkenyloxy, $C_{1-6}$-Alkylthio, $C_{1-6}$-Halogenalkylthio, Cyano, gegebenenfalls substituiertes Phenyl oder gegebenenfalls substituiertes Phenoxy,
$R^2$ Wasserstoff oder Halogen,
$R^3$ Halogen, $C_{1-6}$-Alkyl oder $C_{1-6}$-Alkoxy,
$R^4$ Wasserstoff oder $C_{1-6}$-Alkyl,
$R^5$ und $R^6$ unabhängig voneinander $C_{1-4}$-Alkyl oder zusammen mit dem Stickstoffatom, an das sie geknüpft sind, eine Morpholino- oder Thiomorpholinogruppe und
n 1, 2 oder 3 bedeuten,
dadurch gekennzeichnet dass man

a) ein Keton der allgemeinen Formel

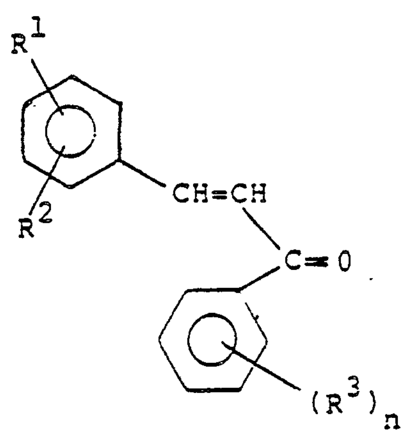

II

worin $R^1$, $R^2$, $R^3$ und n die oben angegebenen Bedeutungen besitzen,
mit einem Phosphonoalkansäureamid der allgemeinen Formel

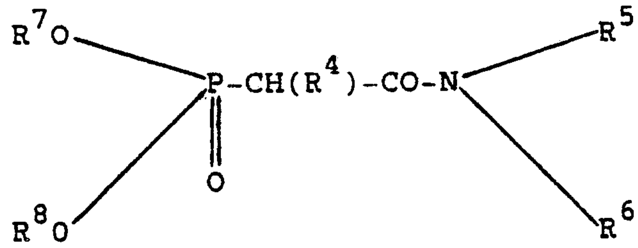

III

worin $R^4$, $R^5$ und $R^6$ die oben angegebenen Bedeutungen besitzen und $R^7$ und $R^8$ unabhängig voneinander eine Niederalkylgruppe oder Arylgruppe bedeuten,
umsetzt, oder

b) eine Acrylsäure der allgemeinen Formel

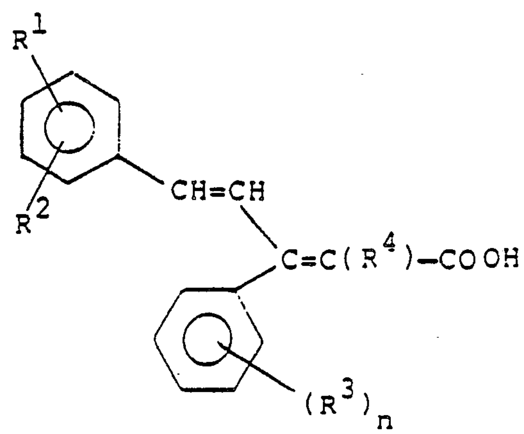

IV

worin $R^1$, $R^2$, $R^3$, $R^4$ und n die oben angegebenen Bedeutungen besitzen,
oder ein reaktionsfähiges Derivat davon mit einem Amin der allgemeinen Formel

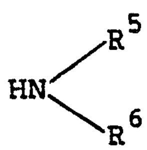

V

worin $R^5$ und $R^6$ die oben angegebenen Bedeutungen besitzen,
oder mit einem reaktionsfähigen Derivat davon umsetzt.

9. Verfahren zur Herstellung eines fungiziden Mittels, dadurch gekennzeichnet, dass man mindestens eine der in Anspruch 1 genannten Verbindungen mit Formulierungshilfsstoffen vermischt.

10. Verfahren zur Bekämpfung von Fungi in der Landwirtschaft und im Gartenbau, dadurch gekennzeichnet, dass man das zu schützende Gut mit einer wirksamen Menge eines Mittels gemäss einem der Ansprüche 1 bis 5 und 7 behandelt.

11. Verfahren zur Bekämpfung von Fungi in der Landwirtschaft und im Gartenbau, dadurch gekennzeichnet, dass man das zu schützende Gut mit einer wirksamen Menge eines Mittels gemäss Anspruch 6 behandelt.

12. Verwendung eines Mittels gemäss einem der Ansprüche 1 bis 5 und 7 zur Bekämpfung von Fungi in der Landwirtschaft und im Gartenbau.

13. Verwendung eines Mittels gemäss Anspruch 6 zur Bekämpfung von Fungi in der Landwirtschaft und im Gartenbau.

Europäisches Patentamt

# EUROPÄISCHER RECHERCHENBERICHT

Nummer der Anmeldung

EP 88114520.5

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | DE - A1 - 3 308 045 (CELAMERCK GMBH)<br>* Ansprüche 1,4-7; Seiten 11,12 *<br>-- | 1,3-5, 8,10, 11,13, 14 | C 07 D 295/18<br>C 07 C 103/58<br>A 01 N 43/84<br>A 01 N 43/40<br>A 01 N 37/18 |
| A | DE - A1 - 3 306 996 (CELAMERCK GMBH)<br>* Ansprüche 1,5-7; Seite 10, Zeile 3 - Seite 11, Zeile 18 *<br>---- | 1,3-5, 8,10, 11,13, 14 | |
| | | | RECHERCHIERTE SACHGEBIETE (Int. Cl.4) |
| | | | C 07 D 295/00<br>C 07 C 103/00 |

Der vorliegende Recherchenbericht wurde für alle Patentansprüche erstellt.

| Recherchenort | Abschlußdatum der Recherche | Prüfer |
|---|---|---|
| WIEN | 12-12-1988 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82